# EUROPÄISCHE PATENTSCHRIFT

(11) **EP 1 040 838 B1**
(45) Veröffentlichungstag und Bekanntmachung des Hinweises auf die Patenterteilung: **26.03.2003**
(21) Anmeldenummer: 00104208.4
(22) Anmeldetag: 01.03.2000
(51) Int. Cl.: A61K 47/26, A61K 31/445, A61K 9/08

(54) **Stabile wässrige Lösung von 3-(1-oxo-1,3-dihydro-isoindol-2-yl)-piperidin-2,6-dion**
Stable aqueous solution of 3-(1-oxo-1,3-dihydro-isoindol-2-yl)-piperidine-2,6-dione
Solution aqueuse stable de 3-(1-oxo-1,3-dihydroisoindol-2-yl)-piperidine-2,6-dione

(30) Priorität: 31.03.1999 DE 19914621
(43) Veröffentlichungstag der Anmeldung: 04.10.2000
(73) Patentinhaber: Grünenthal GmbH, 52078 Aachen (DE)
(72) Erfinder: Germann, Tieno, Dr., 52134 Herzogenrath (DE); Kugelmann, Heinrich, 52068 Aachen (DE)

(56) Entgegenhaltungen:
- EP-A- 0 908 176
- WO-A-94/20085
- SCHMAHL H-J ET AL: "THE ENANTIOMERS OF THE TERATOGENIC THALIDOMIDE ANALOGUE EM 12 2. CHEMICAL STABILITY STEREOSELECTIVITY OF METABOLISM AND RENAL EXCRETION IN THE MARMOSET MONKEY" TOXICOLOGY LETTERS (AMSTERDAM), Bd. 45, Nr. 1, 1989, Seiten 23-34, XP000929156 ISSN: 0378-4274

## Beschreibung

Die Erfindung betrifft eine parenterale Applikationsform des Thalidomid-Derivates 3-(1-oxo-1,3-dihydroisoindol-2-yl)-piperidin-2,6-dion (EM 12) sowie ein Verfahren zu ihrer Herstellung. Diese Applikationsform kann zur Therapie entzündlicher und hämatologischonkologischer Erkrankungen eingesetzt werden

Die überschießende Bildung der proinflammatorischen Zytokine TNF-α (Tumornekrosefaktor) und Interleukin (IL)-12 durch phagozytäre Zellen (z.B. Monozyten) spielt eine zentrale Rolle in der Pathogenese verschiedener entzündlicher Erkrankungen (Trinchieri 1995. Ann.Rev.Immunol. 13: 251).

Ein Ansatz für die Therapie dieser Erkrankungen besteht in der gezielten Unterdrückung der Bildung dieser proinflammatorischen Zytokine durch Gabe immunmodulierender Wirkstoffe, wie zum Beispiel Dexamethason oder Thalidomid bzw. des Thalidomid-Derivates EM 12. Während Kortikoide wie Dexamethason in injizierbaren Formen vorliegen, ist dies für das immunmodulatorisch wirksame Derivat EM 12 bisher nicht der Fall. Für Thalidomid wurde in DE 197 43 968.3 eine parenterale Applikationsform vorgeschlagen.

Thalidomid hat sich in der Behandlung schwerer aphthöser Stomatitis im Vergleich zu den klassischen Immunsuppressiva als überlegen herausgestellt. Weitere Beispiele von Erkrankungen, in denen Thalidomid eine gute Wirksamkeit zeigte, ohne zu einer generellen Immunsuppression zu führen, sind kutaner Lupus erythematodes, Pyoderma gangränosum und urogenitale Ulcera bei Morbus Behcet sowie histologisch von aphthösen Ulzera nicht abweichende Ulzerationen bei HIV-Infizierten, in denen - anders als bei der Mehrzahl der HIV-assoziierten mukokutanen Läsionen - keine mikrobiellen Erreger nachgewiesen werden können. Diese zum Teil auch die Grösse von Major-Aphthen annehmenden Läsionen können im Unterschied zur Stomatitis aphthosa im gesamten Verdauungstrakt auftreten und bei Lokalisation im Rachenraum oder der Speiseröhre durch ihre Schmerz verursachende Wirkung die Nahrungsaufnahme, aber auch die orale Medikamenten-Einnahme erschweren.

Für schwere Fälle pharyngealer oder ösophagealer Ulzera, bei denen die orale Einnahme erschwert, wenn nicht sogar unmöglich sein kann, und für Fälle HIVassoziierter Pathologie, bei denen schwere Durchfall-Symptomatik die orale Einnahme unberechenbar macht, bietet sich die parenterale Wirkstoffgabe an. Der geringen Wasserlöslichkeit von Thalidomid (Arch. Pharm. 321, 371 (1988)) steht jedoch der parenteralen Applikation dieser Wirkstoffe entgegen. Es fehlte deshalb nicht an Versuchen, wasserlösliche Applikations formen zu entwickeln.

Aus DE 42 11 812 sind wasserlösliche Thalidomidderivate bekannt, die im Vergleich zu Thalidomid eine deutlich höhere Wasserlöslichkeit aufweisen und zur parenteralen Applikation geeignet sind.

Weiterhin wurden für die parenterale Applikation Thalidomid-Prodrugs vorgeschlagen, die im physiologischen pH-Wertbereich wasserlöslich applizierbar und toxikologisch unbedenklich sind (DE 196 13 976). Nachteilig ist, daß beide Arten der o.g. Verbindungen in ihrer Herstellung aufwendiger sind als die Herstellung von Thalidomid.

Da das Thalidomid-Derivat EM 12 hinsichtlich seiner Löslichkeit in wässrigem Medium und seiner Neigung zu spontaner Hydrolyse ähnlich ungunstige Eigenschaften aufweist wie Thalidomid, bestand die der Erfindung zugrundeliegende Aufgabe in der Entwicklung einer wasserlöslichen Applikationsform für dieses immunmodulatorisch wirksame Thalidomid-Derivat. Darüber hinaus soll die zu entwickelnde Applikationsform in wässrig gelöster Form stabil sein und durch unphysiologische physikalisch-chemische Eigenschaften sollen keine toxi-kologischen Wirkungen hervorgerufen werden.

Es wurde gefunden, daß die Herstellung wässriger Lösungen durch die Neigung von EM 12 zur spontanen Hydrolyse nicht praktikabel ist. Liegen die pH-Werte von wässri-gen Lösungen jedoch im Bereich von pH kleiner/gleich 5,5 bleibt die Hydrolyse aus.

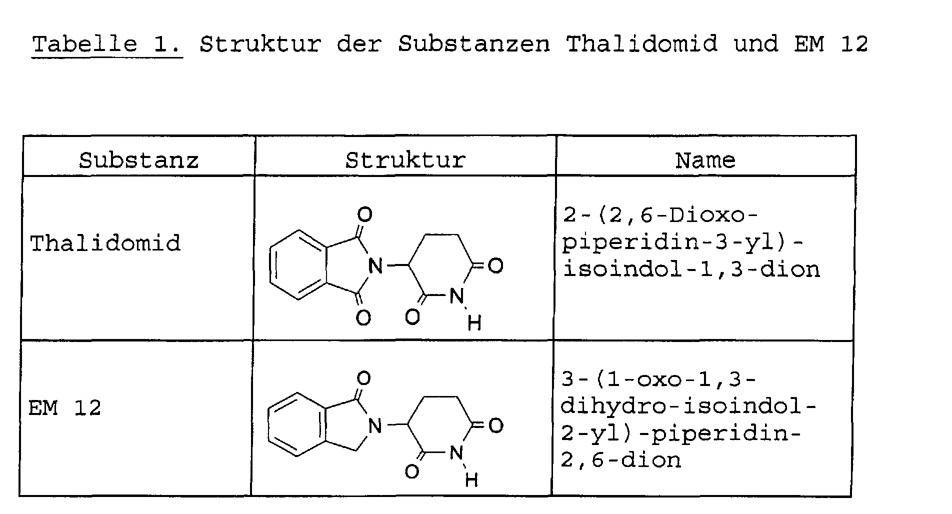

Gegenstand der Erfindung ist dementsprechend eine zur parenteralen Applikation geeignete Lösung von 3-(1-oxo-1,3-dihydro-isoindol-2-yl)-piperidin-2,6-dion (EM 12), wobei diese Lösung eine wässrige mit einem pH-Wert kleiner oder gleich 5,5 ist und als Bestandteil Glukose enthält. Erfindungsgemäß ist EM 12 entweder als Racemat oder in Form eines der Enantiomeren in isotonischer Glukoselösung gelöst. Derartige Lösungen können für die parenterale, insbesondere für die intravenöse, Applikation genutzt werden.

Als injizierbare Applikationsformen von EM 12 eignen sich solche, die einen Wirkstoffgehalt von mindestens 0,2 mg/ml aufweisen.

Weiterer Erfindungsgegenstand ist ein Verfahren zur Herstellung der wässrigen EM-12-Lösung. Dabei wird erfindungsgemäß EM 12 in eine isotonische Glukoselösung mit einem pH-Wert von 4 bis 5 gegeben und diese Mischung bis zur vollständigen Auflösung von EM 12 geschüttelt, und/oder zur Verkürzung der Herstellungszeit anschließend mit Ultraschall behandelt und unter aseptischen Bedingungen filtriert.

Die erfindungsgemäße Applikationsform ist sowohl bei schneller als auch bei langsamer Infusion (10 ml/min) toxikologisch unbedenklich.

Erfindungsgemäße Arzneimittel enthalten neben EM 12 noch Glukose. Gegebenenfalls können der EM 12-Lösung weitere Hilfsstoffe zugesetzt werden. Die Auswahl weiterer Hilfsstoffe sowie die einzusetzenden Mengen hängen davon ab, wie das Arzneimittel genau appliziert werden soll.

Die an den Patienten zu verabreichende Wirkstoffmenge, die vom Gewicht des Patienten, von der parenteralen Applikationsart, der Indikation und dem Schweregrad der Erkrankung abhängt, liegt üblicherweise zwischen 0,1 und 1 mg/kg.

Parenterale EM 12-Lösungen können wie auch Thalidomid-Lösungen zur Therapie von Erkrankungen, bei denen eine überschießende Produktion von TNF-α und IL-12 für die Pathogenese verantwortlich gemacht wird (u.a. Erkrankungen des Darmes, der Haut, der Schleimhäute, der Gefäße sowie von Autoimmunerkrankungen), eingesetzt werden, ferner, aufgrund der anti-angiogenetischen Wirkung auch zur Therapie von hämatologischen Erkrankungen und weiteren onkologischen Erkrankungen.

Zu den Erkrankungen oben genannter Formenkreise zählen unter anderem Entzündungen der Haut (z.B. atopische Dermatitis, Psoriasis, Ekzeme), Entzündungen der Atemwege (z.B. Bronchitis, Pneumonie, Asthma bronchiale, ARDS (adult respiratory distress syndrome), Sarkoidose, Silikose/Fibrose), Entzündungen des Gastrointestinaltraktes (z.B. gastroduodenale Ulcera, Morbus Crohn, ulcerative Colitis), ferner Erkrankungen wie Hepatitis, Pankreatitis, Appendizitis, Peritonitis, Nephritis, Aphthosis, Konjunktivitis, Keratitis, Uveitis, Rhinitis.

Die Autoimmunerkrankungen umfassen z.B. Erkrankungen des arthritischen Formenkreises (z.B. rheumatoide Arthritis, HLA-B27 assoziierte Erkrankungen), ferner Multiple Sklerose, jugendlicher Diabetes oder Lupus erythematosus.

Weitere Indikationen sind Sepsis, bakterielle Meningitis, Kachexie, Transplantat-Abstoßungsreaktionen Graftversus-Host Reaktionen sowie das Reperfusions-syndrom und Atherosklerose.

Ferner gehören hämatologische Erkrankungen wie multiples Myelom und Leukämien sowie weitere onkologische Erkrankungen wie Glioblastom, Prostatacarcinom sowie Mammacarcinom zu den Krankheitsbildern.

### Beispiel

Zur Herstellung von Infusionslösung in einer Konzentration von 200 µg/ml wurden 70 mg racemisches EM 12 in 350 ml einer 5%igen Glukoselösung für Infusionen (pH 4 bis 5) in einer gläsernen Infusionsflasche gegeben. Die Mischung wurde gründlich geschüttelt und 15 Minuten mit Ultraschall behandelt. Da die erreichte gelöste EM 12-Konzentration von der Intensität des Schüttelns und der Ultraschallbehandlung abhängt, wurden beide Schritte bis zur vollständigen Lösung wiederholt. Die Wassertemperatur im Ultraschallbad erreichte maximal 33° C. Die Lösung wurde unter aseptischen Bedingungen durch einen Millex GS Sterilfilter mit 0,22 µm Porengrösse (Millipore S.A., Molsheim, Frankreich) in eine sterile gläserne Infusionsflasche filtriert. Die Lösung wurde bei Raumtemperatur aufbewahrt.

Der pH-Wert der fertigen Lösung betrug 5,5.

### Stabilitätsprüfung

Über einen Zeitraum von 2 Wochen wurden den Lösungen mehrfach Teilmengen zur Analyse entnommen.

Nach 2 Wochen lag das EM 12 noch mit voller biologischer Wirksamkeit vor.

### Nachweis der immunmodulatorisehen Wirksamkeit

Zur Untersuchung der immunmodulatorischen Wirksamkeit der hergestellten Lösungen wurden humane Monozyten aus peripheren Blut-mononucleären Zellen (PBMC) isoliert und mit bakteriellem LPS (Lipopolysaccharid) aktiviert. Die Konzentration von TNF-α und IL-12 in den Zellkulturüberständen wurde mittels Sandwich-ELISAs (Biosource Europe, Fleurus, Belgien) bestimmt..

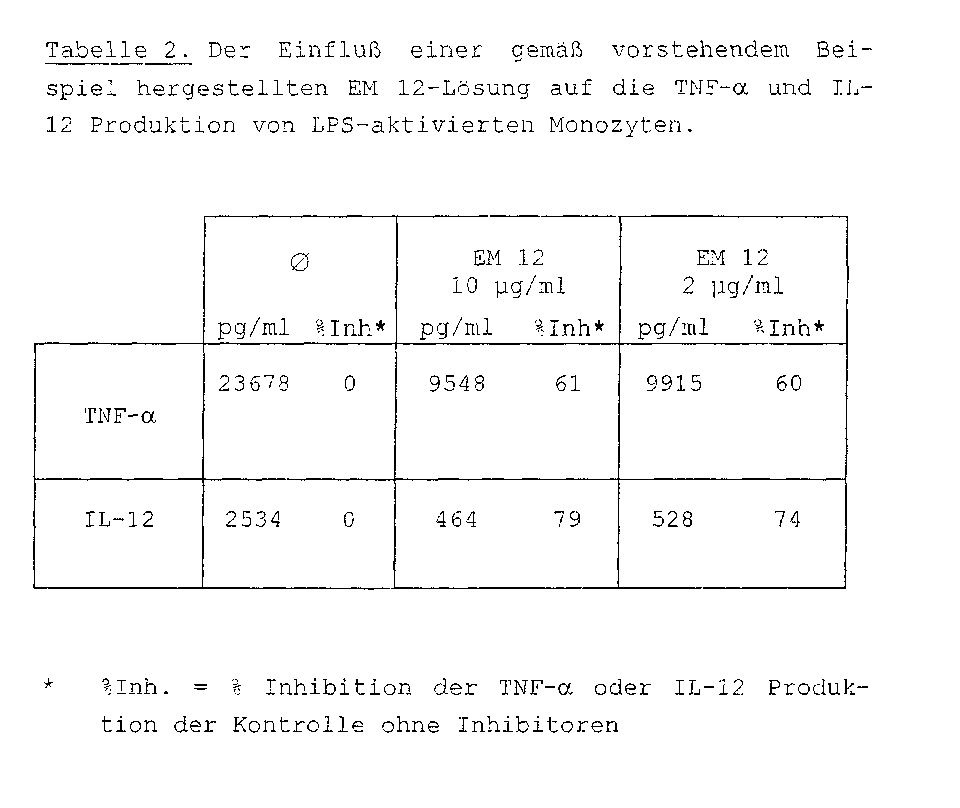

## Patentansprüche

1. Stabile wässrige Lösung enthaltend 3-(1-oxo-1,3-dihydro-isoindol-2-yl)-piperidin-2,6-dion (EM 12) zur parenteralen Verabreichung, wobei das EM 12 in isotonischer Glukoselösung gelöst ist und der pH-Wert der Lösung kleiner oder gleich 5,5 ist.

2. Stabile wässrige Lösung gemäß Anspruch 1, **dadurch gekennzeichnet, daß** sie einen Wirkstoffgehalt von mindestens 0,2 mg/ml aufweist.

3. Verfahren zur Herstellung der stabilen wässrigen Lösung gemäß Anspruch 1, **dadurch gekennzeichnet, daß** 3-(1-oxo-1,3-dihydro-isoindol-2-yl)-piperidin-2,6-dion in eine isotonische Glukoselösung mit einem pH-Wert von 4 bis 5 gegeben und diese Mischung bis zur vollständigen Auflösung geschüttelt, und/oder zur Verkürzung der Herstellungszeit anschließend mit Ultraschall behandelt und unter aseptischen Bedingungen filtriert wird.

## Claims

1. A stable aqueous solution containing 3-(1-oxo-1,3-dihydro-isoindol-2-yl)-piperidine-2,6-dione (EM 12) for parenteral administration, wherein the EM 12 is dissolved in an isotonic glucose solution and the pH of the solution is less than or equal to 5.5.

2. A stable aqueous solution according to claim 1, **characterised in that** it has an active ingredient content of at least 0.2 mg/ml.

3. A method of producing the stable aqueous solution according to claim 1, **characterised in that** 3-(1-oxo-1,3-dihydro-isoindol-2-yl)-piperidine-2,6-dione is added to an isotonic glucose solution with a pH of 4 to 5 and this mixture is shaken until dissolution is complete and/or is subsequently treated with ultrasound in order to shorten the time of production, and is filtered under aseptic conditions.

## Revendications

1. Solution aqueuse stable contenant de la 3-(1-oxo-1,3-dihydroisoindole-2-yl)-pipéridine-2,6-dione (EM 12) destinée à l'administration parentérale, dans laquelle le EM 12 est dissous dans une solution isotonique de glucose et le pH de la solution a une valeur inférieure ou égale à 5,5.

2. Solution aqueuse stable suivant la revendication 1, **caractérisée en ce qu'**elle présente une teneur en substance active d'au moins 0,2 mg/ml.

3. Procédé de préparation d'une solution aqueuse stable suivant la revendication 1, **caractérisé en ce qu'**on incorpore de la 3-(1-oxo-1,3-dihydroisoindole-2-yl)-pipéridine-2,6-dione à une solution isotonique de glucose ayant une valeur de pH de 4 à 5 et on agite ce mélange par secousses jusqu'à dissolution totale, et/ou pour raccourcir le temps de préparation, on traite ensuite le mélange aux ultrasons et on le filtre dans des conditions aseptiques.
